# EUROPEAN PATENT SPECIFICATION

(11) **EP 2 229 921 B1**
(45) Date of publication and mention of the grant of the patent: **12.11.2014**
(21) Application number: 10168449.6
(22) Date of filing: 12.07.2007
(51) Int. Cl.: A61F 2/24, A61F 2/06, B25B 27/10

(54) **Expandable prosthetic valve crimping device**
Crimpvorrichtung für eine erweiterbare Herzklappenprothese
Dispositif de gaufrage de valve prosthétique extensible

(43) Date of publication of application: 22.09.2010
(62) Divisional of application: 07112385.5
(73) Proprietor: Sorin Group Italia S.r.l., 13040 Saluggia (VC) (IT)
(72) Inventor: Righini, Giovanni, 10034, Chivasso (Torino) (IT); Bergamasco, Giovanni, 10137, Torino (IT); Burriesci, Gaetano, London, WC1E 7 JE (GB)
(74) Representative: Bosotti, Luciano

(56) References cited:
- WO-A-2007/030825
- WO-A1-2006/111391
- WO-A2-2008/089365
- US-A1- 2007 027 534

## Description

### TECHNICAL FIELD

The present invention relates to crimping devices for use with implantable devices, such as prosthetic heart valves. Such a device is known, for example, from WO-A-2007/030825.

### BACKGROUND

A wide variety of crimping devices have been developed for crimping stents (e.g., angioplasty stents) onto or within their associated delivery catheters. The term "crimping" is currently used to denote the action of radially contracting an implantable device or a part thereof. A stent for implantation in a body vessel often includes an apertured tubular body that is generally elongated in shape. In other words, the axial length of the stent is larger than, and usually a multiple of, the radial dimension, both in the radially unexpanded and the radially expanded condition of the stent. Many crimping devices known in the art rely on the elongated shape of the stent for proper operation Documents related to crimping include: WO-A-01/21097, WO-A-01/21110, WO-A-2006/117016, WO-A-2005/082578, WO-A-2006/127089, WO-A-2006/088712, WO-A-00/30565, WO-A-01/21103, WO-A-99/53866, WO-A-99/55255, WO-A-99/53866, WO-A-02/092257, EP-A-0 778 009, EP-A-1 353 420, JP-A-11332997A, US-A-5672169, US-A-5947993, US-A-6063102, US-B-6277110, US-B-6309383, US-B-6510722, US-A-2002/035390, US-A-2003/192164, US-A-2004/123437, US-A-2005/166389, US-A-2005/240256, US-A-2005/240256, US-A-2006/265855, WO-A-00/06052, WO-A-00/21464, WO-A-01/21076, WO-A-02/11646, WO-A-2006/136930, US-A-5693066, US-A-5810873, US-A-5951540, US-A-5972016, US-B-6024737, US-B-6051002, US-B-6063102, US-B-6202272, US-B-6277110, US-B-6309383, US-B-6387117, US-B-6481262, US-B-6506201, US-B-6510722, US-B-6726713, and US-A-2007/0061009.

Often implantable devices must be crimped to be coupled to implements or tools for conveying the device to the implantation site.

The crimping action may involve the entire implantable device or only a portion thereof having an annular shape of reduced length (e.g., an axial length that is smaller than a diameter in an expanded condition). Crimping devices known in the art are not ideal for crimping "short" implantable devices, which do not have an axial length much greater than a diameter. These devices may slide or kink sideways with respect to the plane where the crimping action occurs. Likewise, these devices may become unevenly deformed during crimping and thus may be off-center with respect to the desired crimping axis.

### BRIEF DESCRIPTION OF THE DRAWINGS

Figure 1 is a general perspective view of a crimping device as described herein.
Figure 2 is an exploded view showing the basic elements of a device as described herein.
Figure 3a and 3b are schematic representations of the operating principle of a device as described herein.
Figures 4a-4d show a sequence of steps in assembling a device as described herein.
Figures 5 and 6 show an alternative embodiment of a crimping device as described herein.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

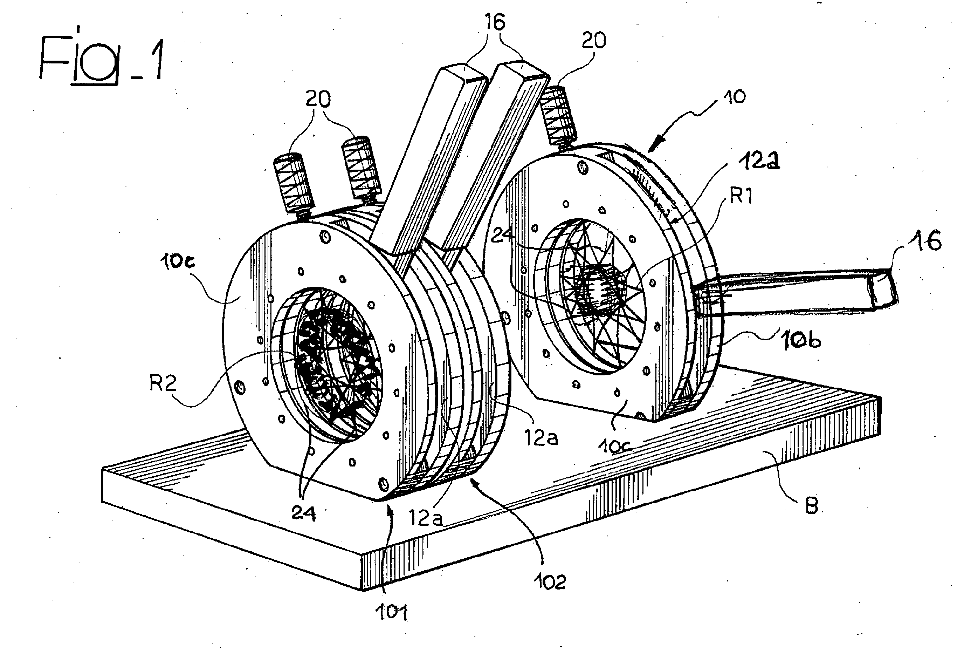

Figure 1 shows a crimping device adapted for use in crimping implantable devices such as, for example, prosthetic heart valves for minimally-invasive (e.g., "sutureless") or percutaneous implantation, according to one embodiment of the present invention. One such exemplary device is disclosed in EP-A-1 690 515. Such a prosthetic heart valve includes an armature with two annular end sections. These annular end sections are "short" elements, having an axial length that is smaller and generally several times smaller than a diameter in the expanded configuration. In some embodiments, the axial length is a submultiple (e.g., 1/5) of the diameter in the expanded configuration. Other exemplary expandable prosthetic heart valves are shown and described in U.S. Publication 2006/0178740 and U.S. Publication 2005/0197695.

The device illustrated in Figure 1 is a multiple crimping tool including a first crimping device 10 adapted for crimping for example, the "inflow" annular end portion R1 of a valve as described in EP-1 690 515, and two "twin" crimping devices 101, 102 adapted for crimping (possibly simultaneously) for example, the "outflow" annular end portion R2 of the same valve. For ease of illustration, Figure 1 shows only the end portions R1 and R2 of the valve in question.

Arranging two units 101, 102 side-by-side for crimping the outflow portion R2 of the valve may be advantageous. In valves such as those disclosed in EP-A-1 690 515, for example, the inflow portion R1 of the valve carries a tubular pericardium structure comprising the prosthetic leaflets of the valve. This structure provides a certain axial stability to the inflow portion R1 of the valve during crimping. Conversely, the outflow portion R2 is comprised essentially of only the valve armature, so thus is may benefit from a crimping action somewhat distributed over its length. Each of the devices 10, 101, and 102 is mounted (e.g., using screws, not visible in the drawing) on a solid base B. The devices 10, 101, 102, the base B, and any related component are comprised of a material suitable for medical use (e.g., polysulfone or Delrin™) and adapted to be easily sterilized.

As shown in Figure 1, the three devices 10, 101, and 102 are substantially identical. Thus, while the description that follows specifically refer to the device 10, it should be understood that this description also applies to the devices 101 and 102. The device shown in Figure 1 may be used during an implantation procedure (i.e., in the operational theatre) to crimp, for example, a prosthetic valve just extracted from its sterile delivery package onto the tool or implement (e.g., a catheter) used for implanting the valve into a patient's body.

According to various embodiments, the device or part subject to crimping is self-expandable. Such a device or part may be constructed of, for example, a superelastic material (e.g., Nitinol), which is crimped from a radially-expanded, "relaxed" condition towards a radially-contracted, "constrained" condition against the elastic force of the device or part. According to other embodiments, the device or part subject to crimping is constructed from a plastically deformable material (e.g., stainless steel), which is plastically deformed from a radially-expanded condition towards a radially-contracted condition, for example, for crimping onto an expandable member such as an inflatable balloon located at or near a distal end of an insertion catheter.

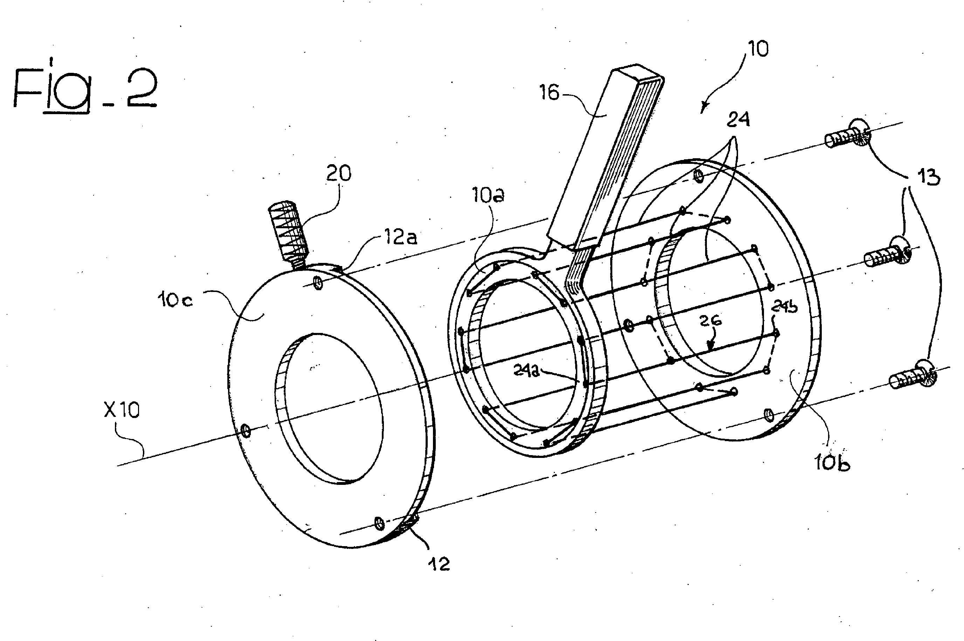

As shown in the exploded view of Figure 2, each of the devices 10, 101, 102 (hereinafter "the device 10") includes two annular bodies 10a, 10b arranged for relative rotation about a common axis X10. A third annular body 10c includes an integral outer circular rim 12 adapted to abut against the outer periphery of the body 10b when the two bodies 10b, 10c are connected to each other via screws 13, with the body 10a interposed therebetween.

The "axial" length (i.e., the length in the direction of the axis X10) and the inner diameter of the rim 12 are selected relative to the thickness and outer diameter of the body 10a in order to ensure radial and axial containment of the body 10a between the two bodies 10b and 10c, while allowing relative rotation of the two bodies 10a, 10b about the axis X10. This relative rotation can be produced by moving a radial arm 16 connected to the body 10a and extending through a slit 12a provided over a given angular length of the rim 12. Either or both of the bodies 10b and 10c are fixed to the base B. Consequently, the arm 16 can be used as an actuating lever to controllably rotate the body 10a with respect to the body 10b.

A screw member or brake member 20 inserted into a threaded radial hole in the rim 12 selectively acts as a brake to fix the body 10a at a given position with respect to the body 10b. Specifically, the brake member 20 is adapted to be loosened and thus radially displaced away from the body 10a to permit free rotation of the body 10a with respect to the bodies 10b and 10c. Conversely, when tightened into the threaded opening, the member 20 advances towards the body 10a to engage the outer periphery thereof and thus prevent rotation of the body 10a around the axis X10.

A screw 13 may be used to couple the bodies 10b and 10c to each other. By removing the screws 13, the body 10a can thus be accessed to remove and replace a linear, wire-like element 26 (e.g., a wire, a suture, a string, a tether, etc.) extending between the bodies 10a and 10b. The wire-like element 26 may include a plurality of crimping elements 24, generally in the form of wire-like formations. The crimping elements 24 are interposed between the two bodies 10a and 10b with each element 24 having a first portion 24a coupled or linked to the body 10a and a second portion 24b linked to the body 10b. As used herein, "linked" is intended to encompass, in addition to a fixed connection, any form of looser association causing the ends 24a, 24b of the elements 24 to follow the respective body 10a, 10b in the relative rotation movement about the axis X10.

In various exemplary embodiments, the body 10b maintains a fixed position with respect to the base B, while the body 10a is selectively and controllably rotated (clockwise, in the example shown) by acting on the "lever" 16. Thus, the ends 24b of the elements 24 will generally retain a fixed or substantially fixed position while the ends 24a will follow the rotation of the body 10a.

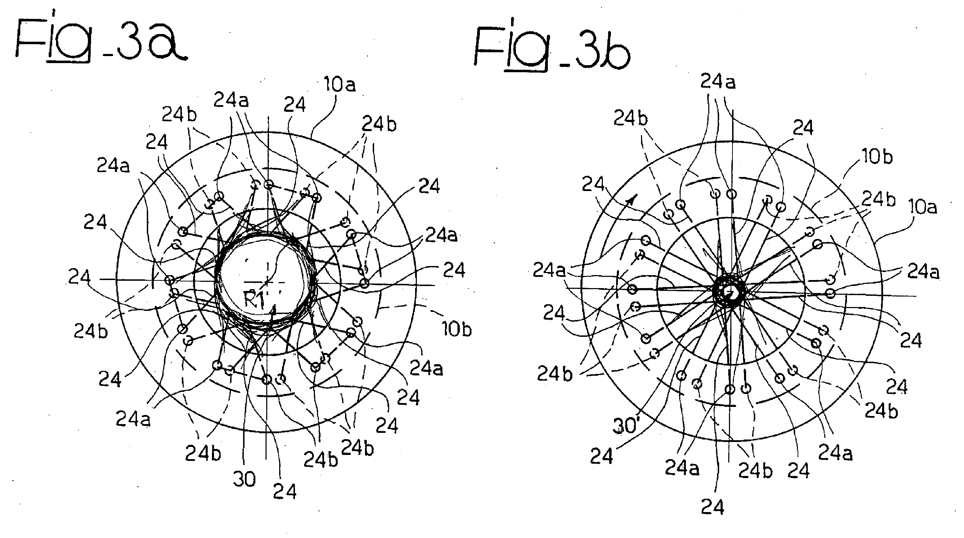

As schematically shown in Figures 3a and 3b, operation of the device 10 relies on the relative rotation of the bodies 10a, 10b, which causes displacement of the elements 24 between a first, "outer" position wherein the ends 24a, 24b of each element jointly define a chordal (i.e., off-center) trajectory with respect to the axis X10, and a second, "inner" position wherein, due to the relative rotation movement of the bodies 10a, 10b, the ends 24a, 24b of each element come to define a substantially diametrical trajectory with respect to the axis X10. This substantially diametrical trajectory extends in the vicinity of the axis X10, but does not cross the axis X10. This substantially diametrical trajectory thus corresponds to a trajectory (much) nearer to the axis X10 than the chordal trajectory.

As a result of a movement between the outer, chordal position shown in Figure 3a and the inner, substantially diametrical position shown in Figure 3b, the distance between the ends 24a, 24b of each element 24 increases. This increase in length can be accommodated in at least three ways, namely: by using elements 24 that are extendable (e.g., elastic), by allowing either or both ends of the elements 24 to be capable of at least slightly sliding with respect to the bodies 10a, 10b, and/or by having the ends 24a, 24b of the elements remain substantially fixed with respect to the bodies 10a, 10b, with the elements 24 (e.g., in the forms of wire-like bodies) extending loosely between the bodies 10a, 10b in the inner chordal position of Figure 3a while becoming increasingly taut when approaching the inner substantially diametrical position of Figure 3b.

The elements 24 comprise an annular array of elements distributed around the axis X10, and the overall result obtainable in passing from the condition illustrated in Figure 3a to the condition illustrated in Figure 3b is similar to operation of an obturator in a camera. In other words, in the outer, chordal position of Figure 3a, the elements 24 jointly define a wider, expanded orifice 30 adapted to receive any of the annular end portions (e.g., the inflow portion R1) of the prosthetic device to be crimped, and in the inner, substantially diametrical position of Figure 3b, the elements 24 jointly define a narrower orifice 30. In this manner, the annular element (e.g., the inflow portion R1), located within the orifice 30 in the position shown in Figure 3a, is radially contracted (and thus "crimped") by the joint action of the elements 24 passing from the position of Figure 3a to the position of Figure 3b.

As described above, an exemplary embodiment provides for the elements 24 being generally loose when in the outer chordal position of Figure 3b. The central orifice 30 defined therebetween will thus be a "soft" orifice adapted to resiliently receive the annular element R1 or R2, while allowing for a certain degree of axial displacement with respect to the central axis X10. Also, being generally loose, the elements 24 will accommodate any irregularities of the outer contour of the element R1 or R2, which may be present if, for example, the element 24 is an apertured, mesh-like body. The elements 24 approaching the inner, substantially diametrical position and becoming increasingly taut will thus have the joint effect of crimping the element R1, R2 while increasingly centering the element R1, R2 about the axis X10 by means of an "isostatic" action.

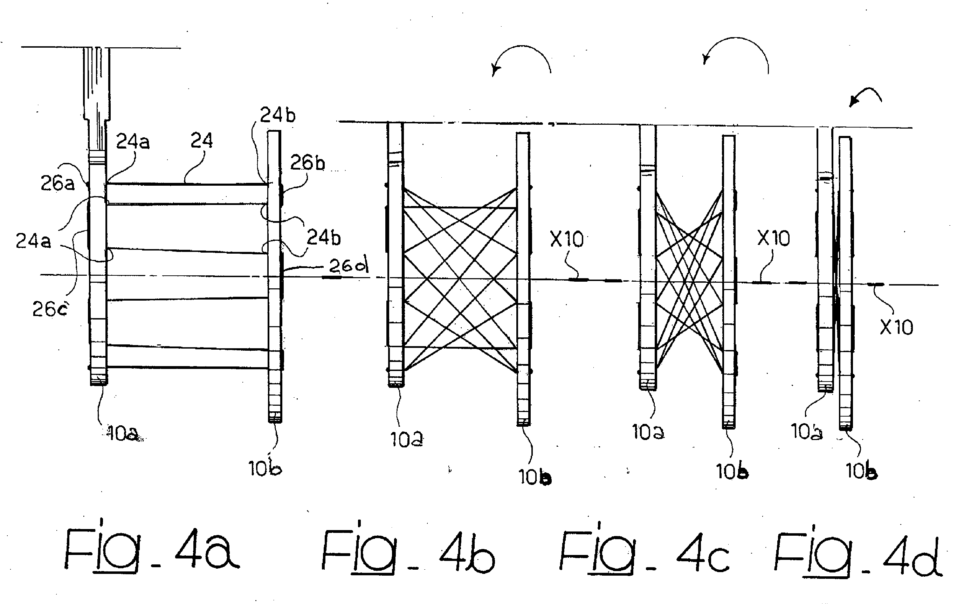

According to various embodiments, the elements 24 are comprised of subsequent sections of one (or more) wire-like members 26 for example, Dacron™ wire which is threaded in a serpentine pattern between the two bodies 10a, 10b. As shown in Figure 4a, by way of example, such a serpentine pattern or trajectory of the wire 26 includes, starting from one end 26a of the wire-like element 26 fixed (e.g., by means of a knot or a stitch) to the body 10a, a first portion extending from the body 10a to the body 10b and comprising a first one of the elements 24 having a first end 24a fixed to the body 10a and the opposite end 24b extending through a hole in (and thus linked to) the body 10b, a short portion 26b extending over the surface of the body 10b opposite the body 10a towards another through hole provided in the body 10b, a second portion extending from the body 10b back to the body 10a and comprising a second one of the elements 24 having an end 24b extending through a hole in (and thus linked to) the body 10b and the opposite end 24a extending through a hole in (and thus linked to) the body 10a, another short portion 26c extending over the surface of the body 10a opposite the body 10b towards another through hole provided in the body 10a, a third portion extending again from the body 10a to the body 10b and comprising a third one of the elements 24 having an end 24a extending through a hole in (and thus linked to) the body 10a and the opposite end 24b extending through a hole in (and thus linked to) the body 10b, another short portion 26d extending over the surface of the body 10b opposite the body 10a towards another through hole provided in the body 10b, and so on. This continues until the "other" end (i.e., the end opposite to the 26a) of the wire-like element 26 is fixed to either of the bodies 10a and 10b. While the holes for the wire 26 in the bodies 10a, 10b will typically constitute two circular crown patterns of equally spaced holes, this is not required. Alternatively, these holes may not be equally angularly spaced and/or may be arranged over plural circular trajectories centered around the axis X10.

As shown in Figures 4a-4d, once the serpentine pattern of the wire 26 is completed, the two bodies 10a, 10b may be rotated, one with respect to the other, about the common axis X10. This rotational movement first causes the portions of the wire element(s) 26 extending between the two bodies 10a, 10b to become skew with respect to the axis X10 thus notionally defining an hourglass-like geometrical surface substantially similar to a rotation hyperboloid. As shown by the sequence of Figures 4a-4d, the "height" of such an hyperboloid (i.e., the distance between the two bodies 10a, 10b) gradually decreases as the rotation movement advances.

When the two bodies 10a, 10b are placed one against each other as shown in Figure 4d, with the wire-like element 26 lying therebetween, the elements 24 are arranged as depicted in Figure 3a (i.e., the elements 24 define a wider, expanded orifice 30). Of course, sequence depicted in Figures 4a-4d is just one exemplary way of obtaining an "obturator-like" arrangement of the elements 24. The arrangement described herein allows the device 10 to be relatively simple and inexpensive. It also allows the device 10 to be a disposable implement for one-time use in the operational theatre to crimp an implantable device.

The brake member 20 is configured to stop the crimping action at any desired position, including intermediate positions. This feature may be useful, for instance, in the multiple crimping arrangement illustrated in Figure 1. First, the operator can crimp a first of annular end portions (e.g., the inflow portion R1). Then, with the inflow portion R1 crimped and safely retained within the device 10 and secured by the respective brake member 20, the operator can crimp the opposite outflow portion R2, using the twin devices 101 and 102. The sequence of operations may also be reversed, such that outflow portion R2 is crimped before inflow portion R1. Figure 1 shows an exemplary situation wherein the inflow end portion R1 has been crimped using the device 10, and the outflow end portion R2 is in the process of being crimped by using the twin devices 101 and 102. This occurs while the device 10 is "locked" by the brake member 20. The operator is thus in a position to fully concentrate on the crimping operation of the outflow portion R2.

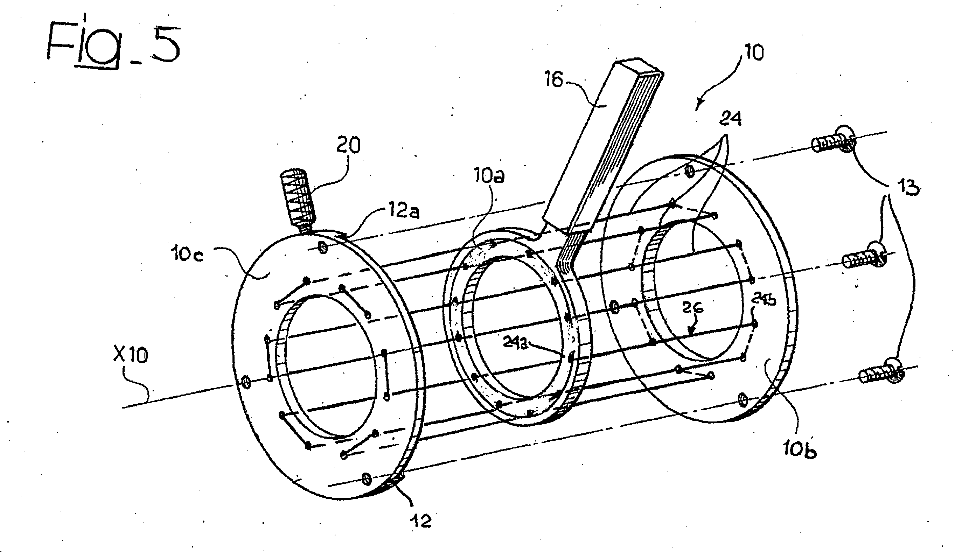

Figure 5 shows an alternative embodiment where the "twin" devices 101, 102 are incorporated to a single, integrated structure. Direct comparison of Figure 5 to Figure 2 shows that the integrated structure of Figure 5 again includes the annular bodies 10a, 10b, 10c, with the body 10a interposed between the bodies 10b, 10c and capable of relative movement with respect to the bodies 10b, 10c. The body 10a can thus be rotated with respect to the bodies 10b and 10c (which are fixed to each other via the screws 13), as a result of actuation of the lever 16. While the embodiment of Figure 2 includes a body 10c that is unperforated (except for the holes provided for the screws 13), in the embodiment of Figure 5, the body 10c includes a "crown" of holes essentially similar to that provided in the body 10b.

In the embodiment of Figure 5, the wire 26 is again imparted a serpentine pattern or trajectory starting, for example, from one end fixed to the body 10b and including: a first portion extending from the body 10b through a hole in the body 10a up to the body 10c, a short portion extending over the surface of the body 10c opposite the body 10a towards another through hole provided in the body 10c, a second portion extending from the body 10c through a hole in the body 10a back to the body 10b, another short portion extending over the surface of the body 10b opposite the body 10a towards another through hole provided in the body 10b, a third portion extending again from the body 10b through a hole in the body 10a up to the body 10c, another short portion extending over the surface of the body 10c opposite the body 10a towards another through hole provided in the body 10b, and so on. This continues until the "other" end of the wire-like element 26 is fixed to any of the bodies 10a, 10b or 10c.

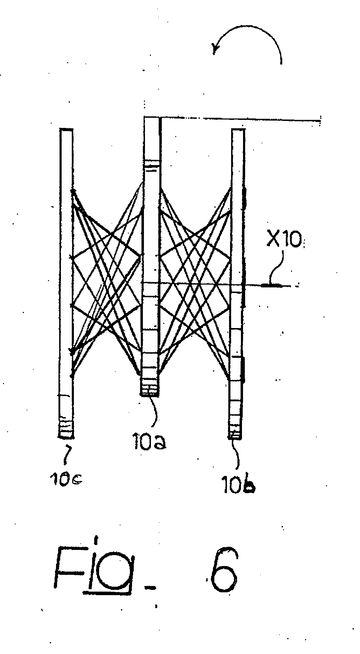

As schematically shown in Figure 6 (which generally corresponds to Figure 4c), once the serpentine pattern of the wire 26 is completed, the body 10a can be rotated with respect to the bodies 10b, and 10c about the common axis X10. As shown, this rotational movement first causes the portions of the wire element(s) 26 extending on either side of the body 10a towards the two bodies 10b and 10c to become skew with respect to the axis X10 thus notionally defining on either side of the body 10a hourglass-like geometrical surfaces substantially similar to a rotation hyperboloid. The "height" of such hyperboloids (i.e. the distance between the body 10a and either of the bodies 10b, 10c) gradually decreases as the rotation movement advances.

When the two bodies 10b, 10c are placed against the body 10a with the wire-like element 26 lying therebetween, the elements 24 are positioned on both sides of the body 10a in an "obturator-like" arrangement or array as shown in Figure 3a. These arrays of elements 24 are arranged side-by-side (e.g., at an axial distance of a few millimeters or less) and can thus jointly co-operate in crimping, for example, the outflow portion R2 of a valve as schematically shown in Figure 1. These two "twin" arrays of elements 24 arranged side-by-side can be operated by acting on the (single) lever 16 of the body 10a and can be locked at any desired crimping position under the action of the brake member 20, which can be selectively operated (e.g., tightened or loosened) to prevent or permit rotation of the body 10a with respect to the bodies 10b and 10c.

Regardless of the embodiment selected, the wire-like characteristic of the elements 24 may be advantageous, as these wire-like elements may easily adapt to an irregular (e.g., V-shaped) outer surface of the device/part to be crimped. Additionally, the wire-like characteristic of the elements 24 may be advantageous in that these elements do not prevent penetration of a sheath-like or cap-like element possibly slid over the crimped device or part to constrain it in the crimped position.

As an alternative to the wire-like configuration, alternative embodiments may include elements 24 in the form of blade-like elements of members (which may be flexible) possibly extending along helical trajectories between the two bodies 10a, 10b. These blade-like elements may be advantageous in more extensively countering any tendency of the device/part being crimped to becoming undesirably kinked during crimping. Also, while in the exemplary embodiments described and shown herein, the bodies 10a, 10b, and 10c are in the form of closed annular bodies, any of them can take the form of an open body (e.g., a sort of "split" ring). By way of example, a flexible element or member can be a wire-like member, a wire, a string, a thread made of natural or synthetic materials, a plastic, a metal, and the like.

In yet another variant of the invention, a device for crimping a heart valve prosthesis onto a delivery system is also provided. Such a device may include multiple crimping modules as shown in Figure 1. Each respective one of the crimping modules is located in a distinct crimping plane one from another. It is appreciated that one crimping module (e.g. 10) can act on the inflow portion of the device, while a second crimping module (e.g. 101, 102) can act, simultaneously or at a different point in time, on the outflow portion of the device. Similarly, each of the modules can be mechanically connected (as shown e.g. in Figures 5 and 6) so that a first module acts on a first portion of the device, while at a predetermined moment in time the other one or more modules act on another portion of the device. By way of further example, three, four, five or more crimping modules act on different portions of the device to crimp it on different sections of the delivery system. In another variant, the crimping planes lie parallel to each other (e.g., in a stacked arrangement). In another variant, the crimping planes interest or lie in a non-parallel relationship to each other.

Consequently, without prejudice to the underlying principles of the invention, details and embodiments may vary, even significantly, with respect to what has been described and illustrated by way of example only, without departing from the scope of the invention as defined by the annexed claims. Likewise, various modifications and additions can be made to the exemplary embodiments discussed without departing from the scope of the present invention. For example, while the embodiments described above refer to particular features, the scope of this invention also includes embodiments having different combinations of features and embodiments that do not include all of the described features.

For instance, in various embodiments, a kit for replacement of a diseased heart valve may be provided, the kit comprising a crimping tool, a heart valve prosthesis deployment tool or portion thereof, an expandable heart valve prosthesis, in which the crimping tool is sized and dimensioned to reduce the size of the heart valve prosthesis to aid in placement of the prosthesis on the heart valve prosthesis deployment tool.

In various embodiments, the kit may further comprise a diseased heart valve leaflet removal tool or portion thereof and a delivery system for guiding the deployment tool and the removal tool into the interior of a heart.

In various embodiments, the kit may comprise a storage container sized and dimensioned to store the heart valve prosthesis in an expanded state.

In various embodiments, a prosthetic heart valve may be provided having an end portion generally disposed within the orifice of the crimping tool.

In various embodiments, the kit may further comprise a solution for maintaining the integrity of the heart valve prosthesis prior to implantation.

In various embodiments, the crimping tool may be configured for uniformly compressing the heart valve prosthesis from an expanded state into a contracted state. In various embodiments, the crimping tool may compress the heart valve prosthesis without damaging leaflets on the heart valve prosthesis. In various embodiments, the crimping tool may be sized and dimensioned to compress the heart valve prosthesis to obtain a compressed heart valve prosthesis, and to deliver the compressed heart valve prosthesis onto the delivery system. In various embodiments, the crimping tool may be variably adjustable to compress and expand the heart valve prosthesis.

In various embodiments, the deployment tool may comprise a guide wire and/or may be reversibly lockable.

In various embodiments, the device for crimping an expandable prosthetic heart valve may comprise a first annular body and a second annular body arranged for relative rotation generally about an axis and an array of linear crimping elements defining an opening having a diameter, wherein rotation of the first body with respect to the second body about the axis causes a change in the diameter.

Accordingly, the scope of the present invention is intended to embrace all such alternatives, modifications, and variations as fall within the scope of the claims, together with all equivalents thereof.

## Claims

1. A kit for replacement of a diseased heart valve, the kit comprising a crimping tool (10), a heart valve prosthesis deployment tool or portion thereof, an expandable heart valve prosthesis (R1, R2), in which the crimping tool (10) is sized and dimensioned to reduce the size of the heart valve prosthesis to aid in placement of the prosthesis on the heart valve prosthesis deployment tool,
wherein the crimping tool (10) for crimping said expandable heart valve prosthesis (R1, R2) comprises a first annular body (10a) and a second annular body (10b) arranged for relative rotation generally about an axis (X10) and an array of linear crimping elements (24) defining an opening (30) having a diameter, wherein rotation of the first body (10a) with respect to the second body (10b) about the axis (X10) causes a change in the diameter,
wherein the crimping elements (24) are interposed between the first annular body (10a) and the second annular body (10b) with each crimping element (24) having a first portion (24a) coupled or linked to the first annular body (10a) and a second portion (24b) linked to the second annular body (10b),
wherein the crimping elements (24) are displaceable between a first position wherein the ends (24a, 24b) of each crimping element jointly define a chordal, off-center, trajectory with respect to said axis (X10), and a second position wherein, upon relative rotation movement of the first and second bodies (10a, 10b), the ends (24a, 24b) of each crimping element come to define a substantially diametrical trajectory with respect to said axis (X10), said substantially diametrical trajectory extending in the vicinity of said axis (X10) without crossing the latter, wherein the kit further comprises the heart valve prosthesis having an end portion (R1, R2) generally disposed within the orifice (30) of the crimping tool (10) and a storage container sized and dimensioned to store the heart valve prosthesis in an expanded state, and
wherein furthermore the crimping elements (24) are generally loose when in said first position so that the orifice (30) defined therebetween is adapted to resiliently receive said end portion (R1, R2) of the heart valve prosthesis.

2. The kit of claim 1 further comprising a diseased heart valve leaflet removal tool or portion thereof and a delivery system for guiding the deployment tool and the removal tool into the interior of a heart.

3. The kit of claim 1 further comprising a solution for maintaining the integrity of the heart valve prosthesis prior to implantation.

4. The kit of claim 1, wherein the crimping tool (10) is variably adjustable to compress and expand the heart valve prosthesis (R1, R2).

5. The kit of claim 1, wherein the deployment tool comprises a guide wire.

6. The kit of claim 1, wherein the deployment tool is reversibly lockable.

## Patentansprüche

1. Ein Kit zum Ersetzen einer kranken Herzklappe, wobei das Kit ein Crimpwerkzeug (10), ein Herzklappenprothesen-Platzierungswerkzeug oder einen Teil davon und eine expandierbare Herzklappenprothese (R1, R2) aufweist, wobei das Crimpwerkzeug (10) ausgelegt und dimensioniert ist, um die Größe der Herzklappenprothese zu reduzieren und so die Platzierung der Prothese an dem Herzklappenprothesen-Platzierungswerkzeug zu unterstützen,
wobei das Crimpwerkzeug (10) zum Crimpen der expandierbaren Herzklappenprothese (R1, R2) einen ersten ringförmigen Körper (10a) und einen zweiten ringförmigen Körper (10b), die für eine relative Rotation allgemein um eine Achse (X10) angeordnet sind, und eine Reihe von linearen Crimpelementen (24), die eine Öffnung (30) mit einem Durchmesser definieren, umfasst, wobei die Rotation des ersten Körpers (10a) gegenüber dem zweiten Körper (10b) um die Achse (X10) eine Durchmesserveränderung bewirkt,
wobei die Crimpelemente (24) zwischen dem ersten ringförmigen Körper (10a) und dem zweiten ringförmigen Körper (10b) angeordnet sind, wobei jedes Crimpelelement (24) einen ersten Bereich (24a), der mit dem ersten ringförmigen Körper (10a) gekoppelt oder verbunden ist, und einen zweiten Bereich (24b), der mit dem zweiten ringförmigen Körper (10b) verbunden ist, aufweist,
wobei die Crimpelemente (24) zwischen einer ersten Position, in welcher die Enden (24a, 24b) von jedem Crimpelement gemeinsam eine chordale, exzentrische Kurve bezüglich der Achse (X10) bilden, und einer zweiten Position, in der nach einer relativen Drehbewegung der ersten und zweiten Körper (10a, 10b) die Enden (24a, 24b) von jedem Crimpelement gegenüber der Achse (X10) eine im Wesentlichen diametrale Kurve bilden, wobei die im Wesentlichen diametrale Kurve sich in der Nähe der Achse (X10) erstreckt, ohne die letztere zu kreuzen,
wobei das Kit weiterhin die Herzklappenprothese, die einen Endbereich (R1, R2) hat, der allgemein innerhalb der Öffnung (30) des Crimpwerkzeugs (10) angeordnet ist, und einen Speicherbehälter, der ausgelegt und dimensioniert ist, um die Herzklappenprothese in einem expandierten Zustand zu lagern, umfasst, und
wobei weiterhin die Crimpelemente (24) allgemein lose sind, wenn sie sich in der ersten Position befinden, so dass die Öffnung (30), die zwischen ihnen definiert wird, geeignet ist, um federnd den Endbereich (R1, R2) der Herzklappenprothese aufzunehmen.

2. Das Kit von Anspruch 1, das weiterhin umfasst ein Beseitigungswerkzeug oder einen Teil davon für einen kranken Herzklappenlappen und ein Zuführsystem, um das Platzierungswerkzeug und das Beseitigungswerkzeug in den Innenraum eines Herzes zu führen.

3. Das Kit von Anspruch 1, welches weiterhin umfasst eine Lösung, um die Integrität der Herzklappenprothese vor der Implantation weiter zu behalten.

4. Das Kit von Anspruch 1, wobei das Crimpwerkzeug (10) variabel einstellbar ist, um die Herzklappenprothese (R1, R1) zu komprimieren und zu expandieren.

5. Das Kit von Anspruch 1, wobei das Platzierungswerkzeug einen Führungsdraht umfasst.

6. Das Kit von Anspruch 1, **dadurch gekennzeichnet, dass** das Platzierungswerkzeug reversibel verriegelbar ist.

## Revendications

1. Kit pour le remplacement d'une valvule cardiaque malade, le kit comprenant un outil de sertissage (10), un outil de déploiement de prothèse de valvule cardiaque ou une partie de celui-ci, une prothèse de valvule cardiaque extensible (R1, R2), dans lequel l'outil de sertissage (10) est calibré et dimensionné pour réduire la taille de la prothèse de valvule cardiaque afin de faciliter le placement de la prothèse sur l'outil de déploiement de prothèse de valvule cardiaque,
dans lequel l'outil de sertissage (10) permettant de sertir ladite prothèse de valvule cardiaque extensible (R1, R2) comprend un premier corps annulaire (10a) et un deuxième corps annulaire (10b) agencés pour effectuer une rotation relative globalement autour d'un axe (X10) et un réseau d'éléments de sertissage linéaires (24) définissant une ouverture (30) ayant un diamètre, dans lequel la rotation du premier corps (10a) par rapport au deuxième corps (10b) autour de l'axe (X10) provoque une variation du diamètre,
dans lequel les éléments de sertissage (24) sont interposés entre le premier corps annulaire (10a) et le deuxième corps annulaire (10b) avec chaque élément de sertissage (24) présentant une première partie (24a) couplée ou liée au premier corps annulaire (10a) et une deuxième partie (24b) liée au deuxième corps annulaire (10b),
dans lequel les éléments de sertissage (24) peuvent se déplacer entre une première position dans laquelle les extrémités (24a, 24b) de chaque élément de sertissage définissent conjointement une trajectoire de corde, excentrée, par rapport audit axe (X10), et une deuxième position dans laquelle, lors d'un mouvement de rotation relatif des premier et deuxième corps (10a, 10b), les extrémités (24a, 24b) de chaque élément de sertissage viennent définir une trajectoire essentiellement diamétrale par rapport audit axe (X10), ladite trajectoire essentiellement diamétrale s'étendant au voisinage dudit axe (X10) sans traverser ce dernier,
dans lequel le kit comprend en outre la prothèse de valvule cardiaque ayant une partie d'extrémité (R1, R2) généralement disposée à l'intérieur de l'orifice (30) de l'outil de sertissage (10) et un récipient de stockage calibré et dimensionné pour stocker la prothèse de valvule cardiaque dans un état dilaté, et
dans lequel en outre les éléments de sertissage (24) sont généralement desserrées lorsqu'ils sont dans ladite première position de sorte que l'orifice (30) défini entre ceux-ci soit adapté à recevoir de manière élastique ladite partie d'extrémité (R1, R2) de la prothèse de valvule cardiaque.

2. Kit de la revendication 1, comprenant en outre un outil d'enlèvement de valvule cardiaque malade ou une partie de celui-ci et un système d'administration pour le guidage de l'outil de déploiement et de l'outil d'enlèvement à l'intérieur du coeur.

3. Kit de la revendication 1, comprenant en outre une solution pour le maintien de l'intégrité de la prothèse de valvule cardiaque avant l'implantation.

4. Kit de la revendication 1, dans lequel l'outil de sertissage (10) peut être réglé de manière variable pour comprimer et dilater la prothèse de valvule cardiaque (R1, R2).

5. Kit de la revendication 1, dans lequel l'outil de déploiement comprend un fil de guidage.

6. Kit de la revendication 1, dans lequel l'outil de déploiement peut être verrouillé de manière réversible.
